(19) Europäisches Patentamt
European Patent Office
Office européen des brevets

(11) **EP 4 603 017 B1**

(12) **EUROPÄISCHE PATENTSCHRIFT**

(45) Veröffentlichungstag und Bekanntmachung des Hinweises auf die Patenterteilung:
**11.03.2026 Patentblatt 2026/11**

(21) Anmeldenummer: **25157399.4**

(22) Anmeldetag: **12.02.2025**

(51) Internationale Patentklassifikation (IPC):
***A61B 5/1455*** *(2006.01)* ***A61B 5/024*** *(2006.01)*

(52) Gemeinsame Patentklassifikation (CPC):
**A61B 5/14552; A61B 5/02416;** A61B 2560/0209

(54) **STEUERUNG EINER LICHTQUELLE EINES PULSOXYMETERS**

CONTROL OF A LIGHT SOURCE OF A PULSOXYMETER

COMMANDE D'UNE SOURCE LUMINEUSE D'UN SPHYGMO-OXYMÈTRE

(84) Benannte Vertragsstaaten:
**AL AT BE BG CH CY CZ DE DK EE ES FI FR GB GR HR HU IE IS IT LI LT LU LV MC ME MK MT NL NO PL PT RO RS SE SI SK SM TR**

(30) Priorität: **13.02.2024 DE 102024104004**

(43) Veröffentlichungstag der Anmeldung:
**20.08.2025 Patentblatt 2025/34**

(73) Patentinhaber: **Löwenstein Medical Technology S.A.**
**2557 Luxembourg (LU)**

(72) Erfinder: **Samie, Farzad**
**76137 Karlsruhe (DE)**

(74) Vertreter: **Löwenstein Medical IP**
**Löwenstein Medical Technology GmbH + Co.KG IP Management**
**Kronsaalsweg 40**
**22525 Hamburg (DE)**

(56) Entgegenhaltungen:
**US-A- 4 063 551 US-A1- 2005 250 997**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 4 603 017 B1

## Beschreibung

### Technisches Gebiet

Die Erfindung betrifft ein Verfahren zum Steuern einer Lichtquelle eines Pulsoxymeters. Des Weiteren betrifft die Erfindung eine Steuereinheit, ein Computerprogramm und ein computerlesbares Medium zum Ausführen des Verfahrens sowie ein Pulsoxymeter.

### Stand der Technik

Ein Pulsoxymeter ist im Allgemeinen eine Vorrichtung zur nicht invasiven Ermittlung der arteriellen Sauerstoffsättigung über die Messung der Lichtabsorption oder Lichtremission beim Durchleuchten von durchblutetem Körpergewebe. Ein solches Pulsoxymeter kann auch zur Pulsfrequenzkontrolle verwendet werden.

Pulsoxymeter werden zunehmend in tragbaren, batteriebetriebenen Anwendungen eingesetzt. So kann ein Pulsoxymeter beispielsweise während eines Notfalltransports an einem Patienten angebracht werden und bei der Verlegung zwischen verschiedenen Krankenhausabteilungen bei ihm verbleiben. Darüber hinaus können Pulsoxymeter als Plug-in-Module für Multiparameter-Patientenmonitore mit begrenztem Energiebudget eingesetzt werden. Solche Anwendungen führen zu einer steigenden Nachfrage nach Pulsoxymetern mit geringerem Stromverbrauch.

US 2005/250997 A1 offenbart ein Pulsoxymeter mit einer Lichtquelle, einem Lichtsensor und einem Optimierer, der Werte von DC- und AC-Komponenten der vom Lichtsensor empfangenen Lichtstrahlen ermitteln und daraus ein Pulsationsverhältnis als ein Verhältnis der AC-Werte zu den DC-Werten berechnen kann. Aus den DC-Werten und den Werten des Pulsationsverhältnisses kann der Optimierer optimierte Stromwerte zum Betreiben der Lichtquelle berechnen.

### Offenbarung der Erfindung

Eine Aufgabe der Erfindung kann darin gesehen werden, ein Verfahren zu schaffen, mit dem der Stromverbrauch eines Pulsoxymeters, insbesondere eines batteriebetriebenen Pulsoxymeters, ohne nennenswerte Qualitätseinbußen verringert werden kann. Eine weitere Aufgabe der Erfindung kann darin gesehen werden, eine Steuereinheit, ein Computerprogramm und ein computerlesbares Medium zum Ausführen eines solchen Verfahrens sowie ein entsprechendes Pulsoxymeter bereitzustellen.

Diese Aufgaben werden durch die Gegenstände der unabhängigen Ansprüche gelöst. Vorteilhafte Ausführungsformen der Erfindung sind in den abhängigen Ansprüchen, der nachfolgenden Beschreibung und den beigefügten Figuren dargelegt.

Ein erster Aspekt der Erfindung betrifft ein Verfahren zum Steuern einer (elektrischen) Lichtquelle eines Pulsoxymeters. Das Pulsoxymeter umfasst zusätzlich zur Lichtquelle einen Lichtsensor, der ausgebildet ist, um einen von einem Körperteil beim Bestrahlen mit Licht von der Lichtquelle transmittierten und/oder reflektierten Lichtanteil in ein (elektrisches) Sensorsignal umzuwandeln. Das Verfahren umfasst: Empfangen des Sensorsignals und eines aktuellen Werts mindestens eines Steuerparameters zum Steuern einer Helligkeit und/oder Farbe der Lichtquelle; Bestimmen eines Skalierungsfaktors aus einem zeitlichen Verlauf einer Amplitude des Sensorsignals unter Berücksichtigung eines Sollwerts für die Amplitude; Bestimmen eines neuen Werts für den mindestens einen Steuerparameter durch Multiplizieren des aktuellen Werts mit dem Skalierungsfaktor; Anwenden des neuen Werts auf den mindestens einen Steuerparameter.

Das Verfahren ermöglicht es, im Betrieb des Pulsoxymeters Strom zu sparen, indem beispielsweise der durch die Lichtquelle fließende Strom, nachstehend Lichtquellenstrom genannt, einerseits verringert wird, solange die Qualität des Photoplethysmogramms (kurz PPG) aus dem Sensorsignal noch ausreichend ist und die erforderliche Genauigkeit noch gegeben ist, und andererseits erhöht wird, falls stärkeres Rauschen auftritt oder allgemein die Qualität des Sensorsignals schlechter wird.

Um eine ausreichende Qualität bei der Bestimmung der Sauerstoffsättigung sicherstellen zu können, sollte die Amplitude des pulsatilen Anteils des Sensorsignals im PPG, auch AC-Anteil genannt, einen bestimmten Wert nicht unterschreiten.

Im Allgemeinen verhält sich die Amplitude des Sensorsignals bei jedem Patienten annähernd linear zum Lichtquellenstrom. Dies liegt daran, dass sich physiologische Faktoren, die die Lichtabsorption von durchblutetem Körpergewebe beeinflussen, wie etwa Fingerdicke oder Hautfarbe, während einer Messung nicht maßgeblich ändern.

Um zu erreichen, dass der AC-Anteil im gewünschten Bereich liegt, kann beispielsweise der Lichtquellenstrom mithilfe des Verfahrens im Betrieb entsprechend angepasst werden. Dabei sollten zu starke und/oder zu häufige Anpassungen vermieden werden.

Das Verfahren kann computerimplementiert sein.

Die Schritte des Verfahrens können im Betrieb des Pulsoxymeters fortlaufend ausgeführt, d. h. zyklisch wiederholt werden. Anders ausgedrückt kann der Wert des mindestens einen Steuerparameters mithilfe des Verfahrens in bestimmten Zeitabständen, beispielsweise von 0,01 s, 0,1 s, 1 s oder 10 s, periodisch aktualisiert werden.

Unter "Sensorsignal" kann ein analoges oder digitales elektrisches Signal verstanden werden. Unter "Amplitude" kann insbesondere eine Amplitude des AC-Anteils des Sensorsignals verstanden werden.

Unter "Steuerparameter" kann beispielsweise einer der folgenden Parameter verstanden werden: ein elektrischer Strom, der durch die Lichtquelle fließt oder fließen soll (beispielsweise zwischen 3 mA und 50 mA); eine elektrische Spannung, die an der Lichtquelle anliegt oder anliegen soll; ein der Lichtquelle vorgeschalteter einstellbarer Vorwiderstand; eine Frequenz, mit der die Lichtquelle ein- und ausgeschaltet wird; ein zeitliches Verhältnis zwischen Einschaltphasen, in denen die Lichtquelle eingeschaltet ist, und Ausschaltphasen, in denen die Lichtquelle ausgeschaltet ist. Beispielsweise kann es sich bei der Frequenz um eine Taktfrequenz und/oder bei dem zeitlichen Verhältnis um einen Tastgrad (englisch *duty cycle*) im Kontext einer Pulsweitenmodulation der Lichtquelle handeln. Bei dem aktuellen Wert des mindestens einen Steuerparameters kann es sich um einen eingestellten und/oder gemessenen und/oder geschätzten Wert handeln.

Unter "Farbe" kann eine Wellenlänge oder ein Wellenlängenbereich eines elektromagnetischen Spektrums verstanden werden. Verschiedene Farben können sich in ihrer Wellenlänge bzw. ihrem Wellenlängenbereich voneinander unterscheiden.

Unter "Sollwert" kann ein fest vorgegebener oder variierbarer gewünschter Wert der Amplitude des Sensorsignals verstanden werden. Beispielsweise kann der variierbare Sollwert im Betrieb abhängig von einer (gemessenen oder geschätzten) Änderung bestimmter physiologischer Faktoren des jeweiligen Patienten variiert werden. Der Sollwert kann beispielsweise zwischen 2 nA und 3 nA, bevorzugt bei 2,5 nA, liegen.

Der neue Wert kann gleich dem Produkt aus dem aktuellen Wert und dem Skalierungsfaktor sein oder ein Wert sein, der basierend auf dem Produkt aus dem aktuellen Wert und dem Skalierungsfaktor bestimmt wurde. Das Anwenden des neuen Werts kann bewirken, dass die Lichtquelle in ihrer Helligkeit und/oder Farbe entsprechend dem neuen Wert (statt des aktuellen Werts) des mindestens einen Steuerparameters angepasst wird.

Ein zweiter Aspekt der Erfindung betrifft eine Steuereinheit. Die Steuereinheit umfasst Mittel, die konfiguriert sind, um das vor- und nachstehend beschriebene Verfahren auszuführen.

Die Mittel können allgemein Hard- und/oder Softwaremodule umfassen. Insbesondere können die Mittel einen Prozessor umfassen, der konfiguriert ist, um das (computerimplementierte) Verfahren auszuführen. Zusätzlich können die Mittel einen Speicher und/oder eine Datenkommunikationsschnittstelle zur drahtlosen und/oder drahtgebundenen Datenkommunikation mit Peripheriegeräten, beispielsweise einem Smartphone, einer Smartwatch, einem Tablet, einem Laptop, einem PC oder einem Beatmungsgerät, umfassen. Alternativ kann die Steuereinheit ausschließlich als Hardware, beispielsweise in Form eines ASIC-Bausteins (ASIC = *application-specific integrated circuit*) oder FPGA-Bausteins (FPGA = *field-programmable gate array*), implementiert sein.

Es wird darauf hingewiesen, dass Merkmale des vor- und nachstehend beschriebenen Verfahrens auch Merkmale der Steuereinheit sein können (und umgekehrt).

Ein dritter Aspekt der Erfindung betrifft ein Pulsoxymeter. Das Pulsoxymeter umfasst eine Lichtquelle, einen Lichtsensor und eine Steuereinheit, wie sie vor- und nachstehend beschrieben wird. Der Lichtsensor ist ausgebildet, um einen von einem Körperteil beim Bestrahlen mit Licht von der Lichtquelle transmittierten und/oder reflektierten Lichtanteil in ein (elektrisches) Sensorsignal umzuwandeln.

Das Pulsoxymeter, beispielsweise dessen Steuereinheit, kann ausgebildet sein, um eine Sauerstoffsättigung ($sO_2$) und/oder einen Puls aus dem Sensorsignal durch Absorptionsspektroskopie zu bestimmen.

Unter "Lichtquelle" kann beispielsweise eine Leuchtdiode, eine Laserdiode, eine Glühlampe oder eine Kombination aus mindestens zwei dieser Beispiele verstanden werden. Insbesondere kann die Lichtquelle ausgebildet sein, um Licht in mindestens zwei voneinander abweichenden vorbestimmten Wellenlängenbereichen, beispielsweise Rotlicht, (Nah-)Infrarotlicht oder Grünlicht, auszusenden. Beispiele für geeignete Wellenlängenbereiche sind 660 nm, 750 nm bis 850 nm und 905 nm bis 940 nm.

Unter "Lichtsensor" kann beispielsweise eine Fotodiode, eine Fotozelle, ein CMOS-Sensor (CMOS = *complementary metal-oxide-semiconductor*), ein CCD-Sensor (CCD = *chargecoupled device*) oder eine Kombination aus mindestens zwei dieser Beispiele verstanden werden.

Unter "Pulsoxymeter" kann auch ein CO-Oxymeter verstanden werden.

Das Pulsoxymeter kann beispielsweise als Clip zur Befestigung am Körperteil, beispielsweise einem Finger, einem Ohrläppchen oder einem Handgelenk, ausgebildet sein.

Das Pulsoxymeter kann so ausgebildet sein, dass die Lichtquelle und der Lichtsensor auf der gleichen Seite des Körperteils und/oder auf einander gegenüberliegenden Seiten des Körperteils angeordnet werden können.

Weitere Aspekte der Erfindung betreffen ein Computerprogramm und ein computerlesbares Medium, auf dem das Computerprogramm gespeichert ist.

Das Computerprogramm umfasst Befehle, die einen Prozessor - beispielsweise einen Prozessor der vor- und nachstehend beschriebenen Steuereinheit - beim Ausführen des Computerprogramms durch den Prozessor veranlassen, das vor- und nachstehend beschriebene Verfahren auszuführen.

Das computerlesbare Medium kann ein flüchtiger oder nicht flüchtiger Datenspeicher sein. Beispielsweise kann das computerlesbare Medium eine Festplatte, ein USB-Speichergerät (USB = *universal serial bus*), ein RAM *(random-access memory)*, ein ROM *(read-only memory)*, ein EPROM *(erasable programmable read-only memory)*, ein EEPROM *(electrically erasable programmable read-only memory)*, ein Flash-Speicher oder eine Kombination aus mindestens zwei dieser Beispiele sein. Das computerlesbare Medium kann auch ein Datenkommunikationsnetzwerk, das das Herunterladen von Programmcode ermöglicht (z. B. über das Internet), oder eine Cloud sein.

Es wird darauf hingewiesen, dass Merkmale des vor- und nachstehend beschriebenen Verfahrens auch Merkmale des Computerprogramms und/oder des computerlesbaren Mediums sein können (und umgekehrt).

Im Folgenden werden verschiedene Ausführungsformen der Erfindung beschrieben. Diese Ausführungsformen sind nicht als Beschränkung des Umfangs der Erfindung zu verstehen.

Gemäß der Erfindung wird ein Mittelwert aus dem zeitlichen Verlauf der Amplitude bestimmt. Der Mittelwert wird dann verwendet, um den Skalierungsfaktor zu bestimmen. Unter "Mittelwert" kann beispielsweise ein arithmetischer, geometrischer, quadratischer oder exponentiell geglätteter Mittelwert verstanden werden. Der Mittelwert kann als ein Qualitätsindex (kurz "QI") in Bezug auf das Sensorsignal aufgefasst werden.

Gemäß einer Ausführungsform kann zum Bestimmen des Skalierungsfaktors ein Quotient aus dem Mittelwert und dem Sollwert gebildet werden. Der Skalierungsfaktor kann gleich dem Quotienten sein oder ein basierend auf dem Quotienten bestimmter Wert sein. Der Quotient kann gebildet werden, indem der Sollwert durch den Mittelwert oder umgekehrt dividiert wird.

Gemäß einer Ausführungsform kann der Mittelwert ein gleitender Mittelwert sein. Der gleitende Mittelwert kann ein einfacher oder gewichteter gleitender Mittelwert sein. Auf diese Weise kann das Sensorsignal wirksam geglättet werden, bevor es weiterverarbeitet wird. Der gleitende Mittelwert wirkt dabei wie ein Tiefpassfilter. Prinzipiell kann der gleitende Mittelwert bestimmt werden, indem das Sensorsignal in mehreren aufeinanderfolgenden Zeitschritten abschnittsweise in einem Fenster abgetastet wird. Dabei kann in jedem der Zeitschritte ein Mittelwert aus den Werten des Sensorsignals im jeweiligen Fenster berechnet werden und das Fenster anschließend so verschoben werden, dass es mit dem Fenster des letzten Zeitschritts teilweise überlappt. Beispielsweise kann das Fenster zwischen (unmittelbar) aufeinanderfolgenden Zeitschritten so verschoben werden, dass der letzte Wert im zuletzt abgetasteten Abschnitt des Sensorsignals aus dem Fenster gestrichen wird und der erste Wert im aktuell abgetasteten Abschnitt des Sensorsignals, d. h. der erste Wert nach dem zuletzt abgetasteten Abschnitt, ins Fenster aufgenommen wird. Der Mittelwert kann dann aus den Werten des derart aktualisierten Fensters erneut berechnet werden. Zusätzlich können die Werte im Fenster in geeigneter Weise gewichtet werden. Das Fenster kann in seiner Breite fest vorgegeben, d. h. konstant, oder variierbar sein, beispielsweise sich von Zeitschritt zu Zeitschritt ändern. Anders ausgedrückt kann das Fenster in einem der Zeitschritte eine andere Breite als in mindestens einem anderen der Zeitschritte haben.

Gemäß der Erfindung wird das Sensorsignal in mehreren aufeinanderfolgenden Zeitschritten empfangen. Der Mittelwert wird in jedem der Zeitschritte unter Verwendung eines Istwerts der Amplitude des im jeweiligen Zeitschritt empfangenen Sensorsignals und unter Verwendung (mindestens) eines früheren Mittelwerts, der in einem dem jeweiligen Zeitschritt (beispielsweise unmittelbar) vorangehenden früheren Zeitschritt bestimmt wurde, bestimmt. Auf diese Weise kann das Sensorsignal wirksam geglättet werden, bevor es weiterverarbeitet wird. Der (gleitende) Mittelwert wirkt dabei wie ein Tiefpassfilter.

Gemäß einer Ausführungsform kann das Verfahren in mehreren aufeinanderfolgenden Zeitschritten ausgeführt werden, wobei in jedem der Zeitschritte das Sensorsignal und der aktuelle Wert empfangen werden können, der Mittelwert, der Skalierungsfaktor und der neue Wert bestimmt werden können und der neue Wert angewandt werden kann.

Gemäß der Erfindung werden der Istwert und der frühere Mittelwert (oder die früheren Mittelwerte) unterschiedlich gewichtet. Dies ermöglicht eine flexible Anpassung der Glättung an unterschiedliche Betriebsbedingungen.

Gemäß einer Ausführungsform kann der Mittelwert in jedem der Zeitschritte entsprechend der folgenden Gleichung bestimmt werden:

$$QI \;=\; \alpha * A \;+\; (1 - \alpha) * QI_{\mathrm{alt}}.$$

Dabei kann *QI* für den Mittelwert im jeweiligen (aktuellen) Zeitschritt, *A* für den Istwert, $QI_{\mathrm{alt}}$ für den früheren Mittelwert und $\alpha$ für einen Gewichtungsfaktor stehen. Der Gewichtungsfaktor kann beispielsweise ein Wert zwischen 0 und 1 sein. Bei dem Gewichtungsfaktor kann es sich insbesondere um einen Erfahrungswert handeln. Der Gewichtungsfaktor kann in jedem der Zeitschritte gleich, d. h. konstant, oder variierbar sein, beispielsweise sich von Zeitschritt zu Zeitschritt ändern. Anders ausgedrückt kann der Gewichtungsfaktor in einem der Zeitschritte vom Gewichtungsfaktor in mindestens einem anderen der Zeitschritte abweichen.

Gemäß einer Ausführungsform kann das Anwenden des neuen Werts umfassen: Bestimmen eines Abweichungswerts, der eine Abweichung des neuen Werts vom aktuellen Wert anzeigt; Bestimmen eines Anpassungswerts unter Verwendung des Abweichungswerts und einer Zuordnungsvorschrift, die möglichen Abweichungswerten jeweils einen Anpassungswert zuordnet; Bestimmen eines angepassten neuen Werts unter Verwendung des aktuellen Werts und des Anpassungswerts, insbesondere durch Addieren des aktuellen Werts und des (positiven oder negativen) Anpassungswerts; Anwenden des angepassten neuen Werts auf den mindestens einen Steuerparameter. Anders ausgedrückt kann der neue Wert in seinem Betrag in geeigneter Weise geändert werden, bevor er auf den mindestens einen Steuerparameter angewandt wird. Dies ermöglicht es beispielsweise, zu große und/oder zu häufige Sprünge bei der Einstellung des mindestens einen Steuerparameters zu vermeiden. Unter "Zuordnungsvorschrift" kann beispielsweise eine mathematische Funktion oder eine Lookup-Tabelle verstanden werden. Die Zuordnungsvorschrift kann beispielsweise in einem Speicher der vor- und nachstehend beschriebenen Steuereinheit hinterlegt sein.

Gemäß einer Ausführungsform kann die Zuordnungsvorschrift eine Sigmoidfunktion sein oder auf einer Sigmoidfunktion basieren. Unter "Sigmoidfunktion" kann allgemein eine Funktion mit einem S-förmigen Graphen verstanden werden. Die Sigmoidfunktion kann zusätzlich einen linearen oder annähernd linearen Abschnitt umfassen. Ein solcher Abschnitt kann unter anderem für die Stabilität des Verfahrens relevant sein.

Gemäß einer Ausführungsform kann die Zuordnungsvorschrift folgendermaßen definiert sein:

$$f(\Delta) = \frac{2 \times S}{1 + 2^{-\Delta}} - S.$$

Dabei kann $f(\Delta)$ für den Anpassungswert, $\Delta$ für den Abweichungswert und $S$ für einen größten zulässigen Betrag des Anpassungswerts stehen. Der Betrag kann im Betrieb des Pulsoxymeters konstant oder variierbar sein, beispielsweise abhängig von den aktuellen Betriebsbedingungen. Der Betrag kann beispielsweise auch als (maximale) Schrittgröße bezeichnet werden.

Gemäß einer Ausführungsform kann eine Approximation $P$ für den Term $2^{-\Delta}$ basierend auf einer Reihenentwicklung, bevorzugt einer Taylorreihe, besonders bevorzugt einer maclaurinschen Reihe, bestimmt werden. In diesem Fall kann die Zuordnungsvorschrift folgendermaßen definiert sein:

$$f(\Delta) = \frac{2 \times S}{1 + P} - S.$$

Dies ermöglicht eine Verbesserung der Recheneffizienz gegenüber Ausführungsformen, wo der Term $2^{-\Delta}$ statt $P$ berechnet wird. Somit kann der Stromverbrauch des Pulsoxymeters weiter verringert werden. Zudem erleichtert dies die Implementierung des Verfahrens als Hard- und/oder Software.

Gemäß einer Ausführungsform kann die Approximation $P$ folgendermaßen definiert sein:

wenn $-\Delta \geq 0$, dann $P = 1 + \sum_{n=1}^{N} \frac{(k \times (-\Delta))^n}{n!}$ und/oder

wenn

$$-\Delta < 0, \text{ dann } P = \frac{1}{1 + \sum_{n=1}^{N} \frac{(-k \times (-\Delta))^n}{n!}}.$$

Dabei kann $N$ für eine vorbestimmte Ordnung der Reihenentwicklung und $k$ für einen vorbestimmten Faktor stehen. Beispielsweise kann $N$ eine natürliche Zahl zwischen 1 und 10, bevorzugt 5, sein und/oder $k$ ein Prozentwert zwischen 0 und 1, bevorzugt zwischen 0,5 und 1,0, besonders bevorzugt 0,75, sein. Bei $N$ und/oder $k$ kann es sich insbesondere um einen Erfahrungswert bzw. Erfahrungswerte handeln.

Dies ermöglicht eine besonders recheneffiziente und/oder besonders einfach zu implementierende Approximation, ohne dass die Genauigkeit des Verfahrens merklich beeinträchtigt wird.

Gemäß einer Ausführungsform kann das Anwenden des neuen Werts umfassen: Bestimmen eines gerundeten Werts aus dem neuen Wert; Anwenden des gerundeten Werts auf den mindestens einen Steuerparameter. Beispielsweise kann der neue Wert entsprechend der Rundungskonvention auf eine oder zwei Dezimalstellen genau gerundet werden. So kann beispielsweise die Grenze für den gerundeten Wert "1" bei 0,5 bis 1,4 bzw. bei 0,45 bis 1,54 liegen. Dies kann die weitere Verarbeitung des neuen Werts vereinfachen. Unter "neuer Wert" kann in diesem Kontext auch ein angepasster neuer Wert, wie er vorstehend beschrieben wird, verstanden werden.

Gemäß einer Ausführungsform kann das Bestimmen des gerundeten Werts umfassen: Verdoppeln des neuen Werts; Runden des verdoppelten Werts; Halbieren des gerundeten verdoppelten Werts. Dies ermöglicht eine relevante Verringerung der Dezimalstellen, ohne dass die Genauigkeit maßgeblich beeinträchtigt wird.

Gemäß einer Ausführungsform kann das Pulsoxymeter ferner eine Batterie zur Stromversorgung mindestens einer elektrischen oder elektronischen Komponente des Pulsoxymeters, insbesondere der Lichtquelle, oder des gesamten Pulsoxymeters umfassen. Dies ermöglicht es, das Pulsoxymeter auch unterwegs zu tragen.

Gemäß einer Ausführungsform kann das Pulsoxymeter ferner eine Anzeigeeinheit zum Anzeigen mindestens eines unter Verwendung des Sensorsignals bestimmten Werts, beispielsweise einer Sauerstoffsättigung oder eines Pulses, umfassen.

## Kurze Beschreibung der Zeichnungen

Im Folgenden werden Ausführungsformen der Erfindung mit Bezug auf die beigefügten Zeichnungen beschrieben. Weder die Beschreibung noch die Zeichnungen sind als Beschränkung des Umfangs der Erfindung zu verstehen.

Fig. 1 zeigt ein Pulsoxymeter gemäß einer Ausführungsform der Erfindung.
Fig. 2 zeigt ein Diagramm, das eine Zuordnungsvorschrift zur Verwendung in einem Verfahren gemäß einer Ausführungsform der Erfindung veranschaulicht.

Die Figuren sind rein schematisch und nicht maßstabsgetreu. Werden in verschiedenen Zeichnungen gleiche Bezugszeichen verwendet, so bezeichnen diese Bezugszeichen gleiche oder gleichwirkende Merkmale.

## Ausführungsformen der Erfindung

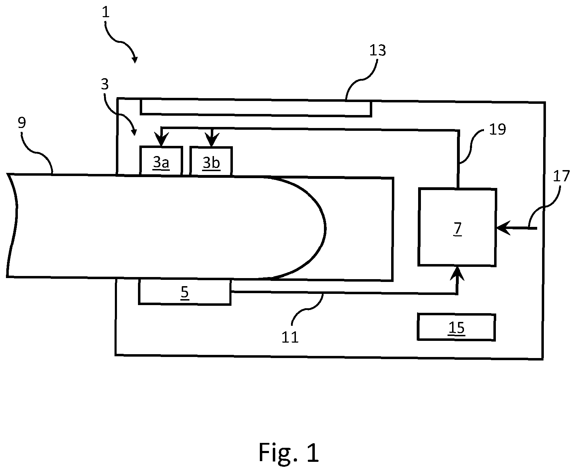

Fig. 1 zeigt ein Pulsoxymeter 1, das eine (elektrische) Lichtquelle 3, einen Lichtsensor 5 und eine Steuereinheit 7 umfasst. Der Lichtsensor 5 ist ausgebildet, um einen von einem Körperteil 9, beispielsweise einem Finger, einem Ohrläppchen oder einem ähnlich dünnen Körperteil, beim Bestrahlen mit Licht von der Lichtquelle 3 transmittierten und/oder reflektierten Lichtanteil in ein (elektrisches) Sensorsignal 11 umzuwandeln.

In diesem Beispiel ist die Steuereinheit 7 ausgebildet, um eine Sauerstoffsättigung ($sO_2$) und/oder einen Puls aus dem Sensorsignal 11 zu bestimmen.

Zusätzlich kann das Pulsoxymeter 1 eine Anzeigeeinheit 13 zum Anzeigen mindestens eines Wertes und/oder mindestens einer Grafik in Bezug auf das Sensorsignal 11, insbesondere in Bezug auf die Sauerstoffsättigung und/oder den Puls, umfassen. Die Anzeigeeinheit 13 kann beispielsweise in Form eines Displays in und/oder an einem Gehäuse des Pulsoxymeters 1 angeordnet sein.

Es ist zweckmäßig, wenn das Pulsoxymeter 1 eine Batterie 15 zur Stromversorgung des Pulsoxymeters 1 umfasst. Somit kann das Pulsoxymeter 1 auch unterwegs getragen werden.

Die Steuereinheit 7 kann allgemein ausgebildet sein, um durch entsprechendes Ansteuern der Lichtquelle 3 die Intensität und/oder Farbe des ausgesandten Lichts zu ändern. In diesem Beispiel umfasst die Lichtquelle 3 eine erste Leuchtdiode 3a zum Aussenden von Licht in einem ersten Wellenlängenbereich, beispielsweise 660 nm, und eine zweite Leuchtdiode 3b zum Aussenden von Licht in einem vom ersten Wellenlängenbereich abweichenden zweiten Wellenlängenbereich, beispielsweise 880 nm bis 940 nm. Die Steuereinheit 7 kann ausgebildet sein, um die Leuchtdioden 3a, 3b im Betrieb des Pulsoxymeters 1 einander abwechselnd ein- und auszuschalten. Alternativ kann die Lichtquelle 3 nur eine Leuchtdiode mit geeignet variierbarem Wellenlängenbereich umfassen. Möglich sind auch andere Typen von Lichtquellen wie Laserdioden oder Glühlampen.

Der Lichtsensor 5 kann beispielsweise eine Fotodiode, eine Fotozelle, einen CMOS-Sensor, einen CCD-Sensor oder eine Kombination aus mindestens zwei dieser Beispiele umfassen.

Das Pulsoxymeter 1 kann beispielsweise als Clip zur Befestigung am Körperteil 9 und/oder als CO-Oxymeter ausgebildet sein.

In diesem Beispiel ist das Pulsoxymeter 1 so ausgebildet, dass die Lichtquelle 3 und der Lichtsensor 5 im Betrieb des Pulsoxymeters 1 auf einander gegenüberliegenden Seiten des Körperteils 9 angeordnet sind. Der Lichtsensor 5 empfängt somit überwiegend den vom Körperteil 9 transmittierten Lichtanteil, wenn die Lichtquelle 3 den Körperteil 9 beleuchtet.

Alternativ kann das Pulsoxymeter 1 so ausgebildet sein, dass die Lichtquelle 3 und der Lichtsensor 5 im Betrieb des Pulsoxymeters 1 auf der gleichen Seite des Körperteils 9 angeordnet sind.

Die Steuereinheit 7 umfasst Mittel, die konfiguriert sind, um ein spezielles Verfahren zum Steuern der Stromversorgung der Lichtquelle 3 auszuführen, wie es nachstehend näher beschrieben wird. Die Mittel können Hard- und/oder Softwaremodule umfassen. Insbesondere können die Mittel einen Speicher und einen Prozessor umfassen. Auf dem Speicher kann ein Computerprogramm gespeichert sein, wobei der Prozessor konfiguriert sein kann, um das Verfahren durch Ausführen des Computerprogramms auszuführen. Zusätzlich können die Mittel eine Datenkommunikationsschnittstelle zur drahtlosen und/oder drahtgebundenen Datenkommunikation mit Peripheriegeräten, beispielsweise einem Smartphone, einer Smartwatch, einem Tablet, einem Laptop, einem PC oder einem Beatmungsgerät, umfassen.

Die Steuereinheit 7 kann auch ausschließlich als Hardware, beispielsweise in Form eines ASIC- oder FPGA-Bausteins, implementiert sein.

Das Verfahren, das computerimplementiert sein kann, umfasst die folgenden Schritte.

In einem ersten Schritt werden in der Steuereinheit 7, beispielsweise in einem entsprechenden Hard- und/oder Softwaremodul der Steuereinheit 7, das Sensorsignal 11 und ein aktueller Wert 17 mindestens eines Steuerparameters zum Steuern der Helligkeit und/oder Farbe der Lichtquelle 3 empfangen. Der mindestens eine Steuerparameter ist in diesem Beispiel ein durch die Lichtquelle 3 fließender elektrischer Strom. Möglich sind aber auch andere Steuerparameter, beispielsweise eine an der Lichtquelle 3 anliegende elektrische Spannung. Der aktuelle Wert 17 kann beispielsweise ein eingestellter und/oder gemessener und/oder geschätzter Wert sein.

In einem zweiten Schritt wird ein Skalierungsfaktor aus einem zeitlichen Verlauf einer Amplitude des Sensorsignals 11 unter Berücksichtigung eines Sollwerts für die Amplitude bestimmt.

In einem dritten Schritt wird ein neuer Wert für den mindestens einen Steuerparameter durch Multiplizieren des aktuellen Werts 17 mit dem Skalierungsfaktor bestimmt. Der neue Wert ist gleich einem Produkt aus dem aktuellen Wert 17 und dem Skalierungsfaktor.

In einem vierten Schritt wird der neue Wert auf den mindestens einen Steuerparameter angewandt. Dabei kann beispielsweise ein Steuersignal 19 zum Steuern der Lichtquelle 3 entsprechend dem neuen Wert erzeugt werden.

Das Verfahren ermöglicht es, im Betrieb des Pulsoxymeters 1 Strom zu sparen, indem beispielsweise der durch die Lichtquelle 3 fließende Strom einerseits verringert wird, solange die Qualität des Photoplethysmogramms (kurz PPG) aus dem Sensorsignal 11 noch ausreichend ist und die erforderliche Genauigkeit noch gegeben ist, und andererseits erhöht wird, falls stärkeres Rauschen auftritt oder allgemein die Qualität des Sensorsignals 11 schlechter wird.

Im zweiten Schritt wird ein Mittelwert $QI$ aus dem zeitlichen Verlauf der Amplitude bestimmt. Der Skalierungsfaktor kann dann unter Verwendung des Mittelwerts $QI$ und des Sollwerts bestimmt werden, insbesondere als Quotient aus dem Mittelwert $QI$ und dem Sollwert.

Mittelwerts $QI$ und des Sollwerts bestimmt werden, insbesondere als Quotient aus dem Mittelwert $QI$ und dem Sollwert.

Das Sensorsignal 11 wird in mehreren aufeinanderfolgenden Zeitschritten empfangen.

Der Mittelwert $QI$ wird in jedem der Zeitschritte unter Verwendung des Istwerts der Amplitude des im jeweiligen Zeitschritt empfangenen Sensorsignals 11 und unter Verwendung (mindestens) eines früheren Mittelwerts $QI_{\mathrm{alt}}$, der in einem dem jeweiligen Zeitschritt (beispielsweise unmittelbar) vorangehenden früheren Zeitschritt bestimmt wurde, bestimmt.

Ein solcher gleitender Mittelwert kann das Sensorsignal 11 wirksam glätten, bevor es weiterverarbeitet wird. Der gleitende Mittelwert wirkt dabei wie ein Tiefpassfilter.

Ein solcher Tiefpassfilter kann sehr einfach implementiert werden, wenn der Mittelwert $QI$ in jedem der Zeitschritte entsprechend der folgenden Gleichung bestimmt wird:

$$QI = \alpha * A + (1 - \alpha) * QI_{\mathrm{alt}}.$$

Dabei steht $A$ für den Istwert der Amplitude im aktuellen Zeitschritt und $\alpha$ für einen Gewichtungsfaktor.

Der Gewichtungsfaktor $\alpha$ kann beispielsweise ein beliebiger Wert zwischen 0 und 1 sein. Der Gewichtungsfaktor $\alpha$ kann in jedem der Zeitschritte gleich, d. h. konstant, oder variierbar sein, beispielsweise sich abhängig von wechselnden Betriebsbedingungen von Zeitschritt zu Zeitschritt ändern. Anders ausgedrückt kann der Gewichtungsfaktor $\alpha$ in einem der Zeitschritte vom Gewichtungsfaktor $\alpha$ in mindestens einem anderen der Zeitschritte abweichen.

Zusätzlich kann der neue Wert, bevor er auf den mindestens einen Steuerparameter angewandt wird, in geeigneter Weise gerundet werden, beispielsweise indem der neue Wert verdoppelt wird, der verdoppelte Wert gerundet wird und der gerundete verdoppelte Wert schließlich halbiert wird.

Der mindestens eine Steuerparameter wird zweckmäßigerweise nur dann auf den neuen Wert gesetzt, wenn dieser signifikant vom aktuellen Wert abweicht. In diesem Fall ist es möglich, dass der Mittelwert $QI$ für den nächsten Zeitschritt gleich dem aktuellen Istwert der Amplitude gesetzt wird, wohingegen der Mittelwert $QI$ andernfalls entsprechend der vorgenannten Gleichung berechnet wird.

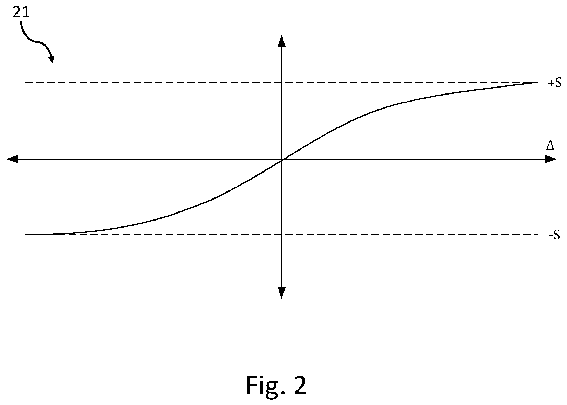

Optional kann ein Abweichungswert $\Delta$, der eine Abweichung des neuen Werts vom aktuellen Wert 17 anzeigt, bestimmt werden, beispielsweise durch Subtrahieren des neuen Werts vom aktuellen Wert 17 (oder umgekehrt). Unter Verwendung des Abweichungswerts $\Delta$ und einer geeigneten Zuordnungsvorschrift 21 (siehe Fig. 2) kann dann ein positiver oder negativer Anpassungswert bestimmt werden. Der Anpassungswert kann verwendet werden, um einen angepassten neuen Wert zu berechnen, beispielsweise durch Addieren des aktuellen Werts 17 und des Anpassungswerts. Der angepasste neue Wert kann dann - als der neue Wert - auf den mindestens einen Steuerparameter angewandt werden.

Die Zuordnungsvorschrift 21 kann beispielsweise in Form einer mathematischen Funktion oder einer Lookup-Tabelle im Speicher der Steuereinheit 7 hinterlegt sein.

Insbesondere kann es sich bei der Zuordnungsvorschrift 21 um eine auf einer Sigmoidfunktion basierende mathematische Funktion handeln. Eine solche Funktion kann beispielsweise folgendermaßen definiert sein:

$$f(\Delta) = \frac{2 \times S}{1 + 2^{-\Delta}} - S,$$

wobei $f(\Delta)$ für den Anpassungswert und $S$ für einen größten zulässigen (im Betrieb des Pulsoxymeters 1 variierbaren oder konstanten) Betrag des Anpassungswerts oder eine maximale Schrittgröße steht. Dadurch können unerwünschte Schwankungen und/oder Sprünge des Werts des mindestens einen Steuerparameters (und damit der Amplitude des Sensorsignals 11) zwischen aufeinanderfolgenden Zeitschritten vermieden werden.

Um den Rechenaufwand, insbesondere bei der Verwendung eines Mikrocontrollers in der Steuereinheit 7, zu verringern, kann als Option eine Approximation P statt des Terms $2^{-\Delta}$ in der Funktion berechnet werden, sodass diese lautet:

$$f(\Delta) = \frac{2 \times S}{1 + P} - S.$$

Die Approximation kann beispielsweise entsprechend der folgenden Gleichung durchgeführt werden:

$$2^{-\Delta} \approx apprx(-\Delta) = \begin{cases} 1 + \sum_{n=1}^{N} \frac{\left(k \times (-\Delta)\right)^n}{n!}, & -\Delta \geq 0 \\ \dfrac{1}{1 + \sum_{n=1}^{N} \frac{\left(-k \times (-\Delta)\right)^n}{n!}}, & -\Delta < 0 \end{cases}$$

Dabei steht $N$ für eine vorbestimmte Ordnung der Reihenentwicklung und $k$ für einen vorbestimmten Faktor. Beispielsweise kann $N$ eine natürliche Zahl zwischen 1 und 10, bevorzugt 5, sein und/oder $k$ ein Prozentwert zwischen 0 und 1, bevorzugt zwischen 0,5 und 1,0, besonders bevorzugt 0,75, sein. Die Zahl $N$ beeinflusst die Genauigkeit der Approximation und den Rechenaufwand. Mit $N = 5$ kann beispielsweise ein guter Kompromiss zwischen Genauigkeit und Rechenaufwand erzielt werden.

Eine solche Reihenentwicklung kann auch als maclaurinsche Reihe bezeichnet werden. Dies ermöglicht eine besonders recheneffiziente und/oder besonders einfach zu implementierende Approximation, ohne dass die Genauigkeit des Verfahrens merklich beeinträchtigt wird.

Wie in Fig. 2 zu erkennen, kann die Zuordnungsvorschrift 21 einen längeren (annähernd) linearen Abschnitt um den Nullpunkt umfassen, was für die Stabilität der Berechnung wichtig ist.

Abschließend wird darauf hingewiesen, dass Begriffe wie "aufweisen", "umfassen", "einschließen", "mit" usw. keine anderen Elemente oder Schritte ausschließen und unbestimmte Artikel wie "ein" oder "eine" keine Vielzahl ausschließen.

Ferner wird darauf hingewiesen, dass Merkmale oder Schritte, die mit Verweis auf eine der vorstehenden Ausführungsformen beschrieben sind, auch in Kombination mit Merkmalen oder Schritten, die mit Verweis auf andere der vorstehenden Ausführungsformen beschrieben sind, verwendet werden können.

Bezugszeichen in den Ansprüchen sind nicht als Beschränkung des Umfangs des durch die Ansprüche definierten Gegenstands zu verstehen.

**Liste der Bezugszeichen**

1    Pulsoxymeter
3    Lichtquelle
3a   erste Leuchtdiode

3b     zweite Leuchtdiode
5      Lichtsensor
7      Steuereinheit
9      Körperteil
11    Sensorsignal
13    Anzeigeeinheit
15    Batterie
17    aktueller Wert
19    Steuersignal
21    Zuordnungsvorschrift
S      größter zulässiger Betrag
Δ      Abweichungswert

**Patentansprüche**

1. Verfahren zum Steuern einer Lichtquelle (3) eines Pulsoxymeters (1), wobei das Pulsoxymeter (1) zusätzlich zur Lichtquelle (3) einen Lichtsensor (5) umfasst, der ausgebildet ist, um einen von einem Körperteil (9) beim Bestrahlen mit Licht von der Lichtquelle (3) transmittierten und/oder reflektierten Lichtanteil in ein Sensorsignal (11) umzuwandeln, wobei das Verfahren umfasst:

Empfangen des Sensorsignals (11) und eines aktuellen Werts (17) mindestens eines Steuerparameters zum Steuern einer Helligkeit und/oder Farbe der Lichtquelle (3), wobei das Sensorsignal (11) in mehreren aufeinanderfolgenden Zeitschritten empfangen wird;
Bestimmen eines Skalierungsfaktors aus einem zeitlichen Verlauf einer Amplitude des Sensorsignals (11) unter Berücksichtigung eines Sollwerts für die Amplitude, wobei ein Mittelwert aus dem zeitlichen Verlauf der Amplitude bestimmt wird und der Skalierungsfaktor unter Verwendung des Mittelwerts bestimmt wird, wobei der Mittelwert in jedem der Zeitschritte unter Verwendung eines Istwerts der Amplitude des im jeweiligen Zeitschritt empfangenen Sensorsignals (11) und unter Verwendung eines früheren Mittelwerts, der in einem dem jeweiligen Zeitschritt vorangehenden früheren Zeitschritt bestimmt wurde, bestimmt wird, wobei der Istwert und der frühere Mittelwert beim Bestimmen des Mittelwerts unterschiedlich gewichtet werden;
Bestimmen eines neuen Werts für den mindestens einen Steuerparameter durch Multiplizieren des aktuellen Werts (17) mit dem Skalierungsfaktor;
Anwenden des neuen Werts auf den mindestens einen Steuerparameter.

2. Verfahren nach Anspruch 1,
wobei zum Bestimmen des Skalierungsfaktors ein Quotient aus dem Mittelwert und dem Sollwert gebildet wird.

3. Verfahren nach einem der vorhergehenden Ansprüche,
wobei der Mittelwert ein gleitender Mittelwert ist.

4. Verfahren nach einem der vorhergehenden Ansprüche,

wobei der Mittelwert in jedem der Zeitschritte entsprechend der folgenden Gleichung bestimmt wird:

$$QI = \alpha * A + (1-\alpha) * QI_{\mathrm{alt}};$$

wobei $QI$ für den Mittelwert im jeweiligen Zeitschritt, $A$ für den Istwert, $QI_{\mathrm{alt}}$ für den früheren Mittelwert und $\alpha$ für einen Gewichtungsfaktor steht.

5. Verfahren nach einem der vorhergehenden Ansprüche,
wobei das Anwenden des neuen Werts umfasst:

Bestimmen eines Abweichungswerts (Δ), der eine Abweichung des neuen Werts vom aktuellen Wert (17) anzeigt;
Bestimmen eines Anpassungswerts unter Verwendung des Abweichungswerts (Δ) und einer Zuordnungsvorschrift (21), die möglichen Abweichungswerten jeweils einen Anpassungswert zuordnet;
Bestimmen eines angepassten neuen Werts unter Verwendung des aktuellen Werts (17) und des Anpassungswerts, insbesondere durch Addieren des aktuellen Werts (17) und des Anpassungswerts;
Anwenden des angepassten neuen Werts auf den mindestens einen Steuerparameter.

6. Verfahren nach Anspruch 5,

wobei die Zuordnungsvorschrift (21) eine Sigmoidfunktion ist oder auf einer Sigmoidfunktion basiert; und/oder
wobei die Zuordnungsvorschrift (21) folgendermaßen definiert ist:

$$f(\Delta) = \frac{2 \times S}{1 + 2^{-\Delta}} - S,$$

wobei $f(\Delta)$ für den Anpassungswert, $\Delta$ für den Abweichungswert $(\Delta)$ und $S$ für einen größten zulässigen Betrag $(S)$ des Anpassungswerts steht.

7. Verfahren nach Anspruch 6,
wobei eine Approximation $P$ für den Term $2^{-\Delta}$ basierend auf einer Reihenentwicklung, bevorzugt einer Taylorreihe, besonders bevorzugt einer maclaurinschen Reihe, bestimmt wird und die Zuordnungsvorschrift (21) folgendermaßen definiert ist:

$$f(\Delta) = \frac{2 \times S}{1 + P} - S.$$

8. Verfahren nach Anspruch 7,

wobei die Approximation $P$ folgendermaßen definiert ist:
wenn

$$-\Delta \geq 0, \text{ dann } P = 1 + \sum_{n=1}^{N} \frac{(k \times (-\Delta))^n}{n!}$$

und/oder
wenn

$$-\Delta < 0, \text{ dann } P = \frac{1}{1 + \sum_{n=1}^{N} \frac{(-k \times (-\Delta))^n}{n!}};$$

wobei $N$ für eine vorbestimmte Ordnung der Reihenentwicklung und $k$ für einen vorbestimmten Faktor steht.

9. Steuereinheit (7), umfassend Mittel, die konfiguriert sind, um das Verfahren nach einem der vorhergehenden Ansprüche auszuführen.

10. Pulsoxymeter (1), umfassend:

eine Lichtquelle (3);
einen Lichtsensor (5), der ausgebildet ist, um einen von einem Körperteil (9) beim Bestrahlen mit Licht von der Lichtquelle (3) transmittierten und/oder reflektierten Lichtanteil in ein Sensorsignal (11) umzuwandeln;
eine Steuereinheit (7) nach Anspruch 9.

11. Computerprogramm, umfassend Befehle, die einen Prozessor beim Ausführen des Computerprogramms durch den Prozessor veranlassen, das Verfahren nach einem der Ansprüche 1 bis 8 auszuführen.

12. Computerlesbares Medium, auf dem das Computerprogramm nach Anspruch 11 gespeichert ist.

**Claims**

1. A method for controlling a light source (3) of a pulse oximeter (1), wherein the pulse oximeter (1) comprises, in addition to the light source (3), a light sensor (5) that is designed to convert a portion of light transmitted and/or reflected by a body part (9), when same is irradiated with light from the light source (3), into a sensor signal (11), wherein the method comprises:

   receiving the sensor signal (11) and a current value (17) of at least one control parameter for controlling a brightness and/or color of the light source (3), wherein the sensor signal (11) is received in a plurality of successive time steps;
   determining a scaling factor from a time profile of an amplitude of the sensor signal (11) taking into account a target value for the amplitude, wherein a mean value is determined from the time profile of the amplitude and the scaling factor is determined using the mean value, wherein the mean value is determined in each of the time steps using an actual value of the amplitude of the sensor signal (11) received in the relevant time step and using a previous mean value determined in an earlier time step preceding the relevant time step, wherein the actual value and the previous mean value are weighted differently when determining the mean value;
   determining a new value for the at least one control parameter by multiplying the current value (17) by the scaling factor;
   applying the new value to the at least one control parameter.

2. The method according to claim 1,
   wherein a quotient is formed from the mean value and the target value in order to determine the scaling factor.

3. The method according to one of the preceding claims,
   wherein the mean value is a moving mean value.

4. The method according to one of the preceding claims,

   wherein the mean value is determined in each of the time steps according to the following equation:

   $$QI = \alpha * A + (1 - \alpha) * QI_{\text{old}};$$

   wherein $QI$ denotes the mean value in the relevant time step, $A$ denotes the actual value, $QI_{\text{old}}$ denotes the previous mean value, and $\alpha$ denotes a weighting factor.

5. The method according to one of the preceding claims,
   wherein applying the new value comprises:

   determining a deviation value ($\Delta$) indicating a deviation of the new value from the current value (17);
   determining an adjustment value using the deviation value ($\Delta$) and an assignment rule (21) which assigns a relevant adjustment value to possible deviation values;
   determining an adjusted new value using the current value (17) and the adjustment value, in particular by adding the current value (17) and the adjustment value;
   applying the adjusted new value to the at least one control parameter.

6. The method according to claim 5,

   wherein the assignment rule (21) is a sigmoid function or is based on a sigmoid function; and/or
   wherein the assignment rule (21) is defined as follows:

   $$f(\Delta) = \frac{2 \times S}{1 + 2^{-\Delta}} - S,$$

   wherein $f(\Delta)$ denotes the adjustment value, $\Delta$ denotes the deviation value ($\Delta$), and $S$ denotes a maximum permissible magnitude ($S$) of the adjustment value.

7. The method according to claim 6,

wherein an approximation $P$ for the term $2^{-\Delta}$ is determined based on a series expansion, preferably a Taylor series, particularly preferably a Maclaurin series, and the assignment rule (21) is defined as follows:

$$f(\Delta) = \frac{2 \times S}{1+P} - S.$$

8. The method according to claim 7,

   wherein the approximation $P$ is defined as follows:
   if

$$-\Delta \geq 0, \text{ then } P = 1 + \sum_{n=1}^{N} \frac{(k \times (-\Delta))^n}{n!}$$

   and/or
   if

$$-\Delta < 0, \text{ then } P = \frac{1}{1 + \sum_{n=1}^{N} \frac{(-k \times (-\Delta))^n}{n!}};$$

   wherein $N$ denotes a predetermined order of the series expansion and $k$ denotes a predetermined factor.

9. A control unit (7), comprising means that are configured to carry out the method according to one of the preceding claims.

10. A pulse oximeter (1), comprising:

    a light source (3);
    a light sensor (5) that is designed to convert a portion of light transmitted and/or reflected by a body part (9), when same is irradiated with light from the light source (3), into a sensor signal (11);
    a control unit (7) according to claim 9.

11. A computer program, comprising commands which, when the computer program is executed by a processor, cause the processor to carry out the method according to one of claims 1 to 8.

12. A computer-readable medium on which the computer program according to claim 11 is stored.

**Revendications**

1. Procédé de commande d'une source lumineuse (3) d'un oxymètre de pouls (1), dans lequel l'oxymètre de pouls (1) comprend, en plus de la source lumineuse (3), un capteur de lumière (5) configuré pour convertir une composante lumineuse transmise et/ou réfléchie par une partie de corps (9) lors de l'irradiation avec de la lumière provenant de la source lumineuse (3) en un signal de capteur (11), le procédé comprenant :

   la réception du signal de capteur (11) et d'une valeur actuelle (17) d'au moins un paramètre de commande pour commander une luminosité et/ou une couleur de la source lumineuse (3), ledit signal de capteur (11) étant reçu en plusieurs pas de temps successifs ;
   la détermination d'un facteur d'échelle à partir d'une évolution temporelle d'une amplitude du signal de capteur (11), en tenant compte d'une valeur de consigne pour l'amplitude, dans lequel une valeur moyenne est déterminée à partir de l'évolution temporelle de l'amplitude et le facteur d'échelle est déterminé à l'aide de la valeur moyenne, dans lequel la valeur moyenne est déterminée, à chacun des pas de temps, à l'aide d'une valeur réelle de l'amplitude du signal de capteur (11) reçu au pas de temps respectif et à l'aide d'une valeur moyenne antérieure déterminée lors d'un pas de temps antérieur précédant le pas de temps respectif, dans lequel la valeur réelle et la valeur moyenne antérieure sont pondérées différemment lors de la détermination de la valeur moyenne ;

la détermination d'une nouvelle valeur pour l'au moins un paramètre de commande par multiplication de la valeur actuelle (17) par le facteur d'échelle ;
l'application de la nouvelle valeur à l'au moins un paramètre de commande.

2. Procédé selon la revendication 1,
dans lequel, pour déterminer le facteur d'échelle, un quotient est formé à partir de la valeur moyenne et de la valeur de consigne.

3. Procédé selon l'une des revendications précédentes,
dans lequel la valeur moyenne est une moyenne glissante.

4. Procédé selon l'une des revendications précédentes,

dans lequel, à chaque pas de temps, la valeur moyenne est déterminée selon l'équation suivante :

$$QI = \alpha * A + (1 - \alpha) * QI_{\text{alt}} ;$$

dans lequel $QI$ représente la valeur moyenne au pas de temps respectif, $A$ la valeur réelle, $QI_{\text{alt}}$ la valeur moyenne antérieure et $\alpha$ un facteur de pondération.

5. Procédé selon l'une des revendications précédentes,
dans lequel l'application de la nouvelle valeur comprend :

la détermination d'une valeur d'écart ($\Delta$) indiquant un écart de la nouvelle valeur par rapport à la valeur actuelle (17) ;
la détermination d'une valeur d'ajustement à l'aide de la valeur d'écart ($\Delta$) et d'une règle d'association (21), laquelle associe respectivement une valeur d'ajustement à des valeurs d'écart possibles ;
la détermination d'une nouvelle valeur ajustée à l'aide de la valeur actuelle (17) et de la valeur d'ajustement, en particulier par addition de la valeur actuelle (17) et de la valeur d'ajustement ;
l'application de la nouvelle valeur ajustée à l'au moins un paramètre de commande.

6. Procédé selon la revendication 5,

dans lequel la règle d'association (21) est une fonction sigmoïde ou est basée sur une fonction sigmoïde ; et/ou dans lequel la règle d'association (21) est définie comme suit :

$$f(\Delta) = \frac{2 \times S}{1 + 2^{-\Delta}} - S,$$

dans lequel f($\Delta$) représente la valeur d'ajustement, $\Delta$ la valeur d'écart ($\Delta$) et le montant maximal autorisé ($S$) de la valeur d'ajustement.

7. Procédé selon la revendication 6,
dans lequel une approximation P pour le terme $2^{-\Delta}$ est déterminée sur la base d'un développement en série, de préférence une série de Taylor, de façon particulièrement préférée une série de Maclaurin, et la règle d'association (21) est définie comme suit :

$$f(\Delta) = \frac{2 \times S}{1 + P} - S.$$

8. Procédé selon la revendication 7,

dans lequel l'approximation $P$ est définie comme suit :

si

$$-\Delta \geq 0, \text{ alors } P = 1 + \sum_{n=1}^{N} \frac{\left(k \times (-\Delta)\right)^n}{n!}$$

et/ou
si

$$-\Delta < 0, \text{ alors } P = \frac{1}{1 + \sum_{n=1}^{N} \frac{\left(-k \times (-\Delta)\right)^n}{n!}} \; ;$$

dans lequel $N$ représente un ordre prédéterminé du développement en série et $k$ représente un facteur prédéterminé.

9. Unité de commande (7), comprenant des moyens configurés pour mettre en œuvre le procédé selon l'une des revendications précédentes.

10. Oxymètre de pouls (1), comprenant :

une source lumineuse (3) ;
un capteur de lumière (5) configuré pour convertir une composante lumineuse transmise et/ou réfléchie par une partie de corps (9) lors de l'irradiation avec de la lumière provenant de la source lumineuse (3) en un signal de capteur (11) ;
une unité de commande (7) selon la revendication 9.

11. Programme informatique comprenant des instructions amenant un processeur à mettre en œuvre le procédé selon l'une quelconque des revendications 1 à 8 lors de l'exécution du programme informatique par le processeur.

12. Support lisible par ordinateur, sur lequel est enregistré le programme informatique selon la revendication 11.

Fig. 1

Fig. 2

IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 2005250997 A1